# EUROPEAN PATENT SPECIFICATION

(11) **EP 1 928 428 B1**
(45) Date of publication and mention of the grant of the patent: **01.09.2010**
(21) Application number: 06831860.9
(22) Date of filing: 27.10.2006
(51) Int. Cl.: A61K 9/28, A61K 31/5513

(54) **A PHARMACEUTICAL FORMULATION CONTAINING OLANZAPINE**
OLANZAPIN ENTHALTENDE PHARMAZEUTISCHE ZUSAMMENSETZUNG
COMPOSITION PHARMACEUTIQUE CONTENANT DE L'OLANZAPINE

(30) Priority: 03.11.2005 GB 0522474
(43) Date of publication of application: 11.06.2008
(73) Proprietor: Actavis Group PTC EHF, 220 Hafnarfjordur (IS)
(72) Inventor: KRISTJANSSON, Torfi, 108 Reykjavik (IS)
(74) Representative: Gillard, Richard Edward
(86) International application number: PCT/IB2006/003922
(87) International publication number: WO 2007/052164

(56) References cited:
- EP-A1- 0 733 367
- EP-A1- 0 733 635
- WO-A-2004/058223
- WO-A-2005/009407
- US-A1- 2005 288 276
- ANONYMOUS: "Kollicoat IR" TECNICAL INFORMATION, [Online] July 2006 (2006-07), pages 1-12, XP002429201 Retrieved from the Internet: URL:http://www.pharma-solutions.basf.com/( kuoimf45chh0p345aegl3v45)/pdf/statements/T echnical%20Information/Pharma%20Solutions/ Kollicoat%20IR.pdf> [retrieved on 2007-04-12]
- "Remington's Pharmaceutical Sciences" 1975, MACK PUBLISHING GROUP , EASTON * page 1244 *
- "The Merck Index" 1989, MERCK & CO. INC , RAHWAY * page 7561 *
- KIBBE A.H.: "HANDBOOK OF PHARMACEUTICAL EXCIPIENTS" 2000, AMERICAN PHARMACEUTICAL ASSOCIATION , WASHINGTON DC * pages 424-425, paragraphs 13,19 *

## Description

This invention relates to a pharmaceutical formulation and in particular to a stable composition for a pharmaceutical dosage form containing olanzapine.

Olanzapine (2-methyl-4-(4-methyl-1-piperazinyl)-10H-thieno[2,3-b] [1,5] benzodiazepine) is a psychotropic agent belonging to the class of drugs known as thienobenzodiazepines. Olanzapine is used for the treatment of schizophrenia and is indicated for the prevention of recurrence of manic episodes in patients with bipolar disorder whose manic episode has responded to olanzapine treatment.

US 4,115,568 discloses a general formula of thieno[1,5] benzodiazepines having useful CNS activity. US 5,229,382 discloses olanzapine per se as well its CNS activity.

However, the current commercially available tablets are limited by a high rate of degradation of the drug. Olanzapine is known to suffer from problems associated with the intrinsic nature of olanzapine, e.g. moisture sensitivity, propensity for discoloration, metastability of various crystalline and amorphous forms and degradation after compounding into tablets.

A number of attempts have been made to avoid the degradation of olanzapine. For example, WO 2005/009407 discloses a pharmaceutical composition containing olanzapine particles or powder in which the olanzapine particles or powder have a coating comprising lactose and/or mannitol. This document also discloses a pharmaceutical composition containing olanzapine and suitable excipients in which the olanzapine and excipients have a coating selected from carrageenan, sodium alginate, polyvinyl alcohol-polyethylene glycol graft copolymer, and a titanium dioxide-talc mixture.

There remains, however, a need to provide further and/or improved formulations to avoid degradation and discoloration of olanzapine formulations.

WO 2005/009407 relates to a olanzapine formulation stable to discolouration. The pharmaceutical formulation includes olanzapine particles or powder, and a coating on the olanzapine particles or powder.

EP 0 733 635 relates to a stable polymorph of olanzapine.

EP 0 733 367 relates to a solid oral formulation of olanzapine and a process for making such formulation. The formulation comprises olanzapine intimately mixed with a bulking agent, a binder, a disintegrant, a dry binder and lubricant.

Accordingly, the present invention provides a pharmaceutical composition which is a tablet comprising olanzapine or a pharmaceutically acceptable salt thereof, and one or more suitable pharmaceutical excipients, wherein the tablet is coated with a coating comprising polyvinyl alcohol which consists of vinyl alcohol/acetate monomers.

It has been found that surprisingly a coating containing polyvinyl alcohol reduces the rate of degradation of olanzapine when compounded into tablets.

The coating of the present invention contains polyvinyl alcohol which is typically partially hydrolysed (e.g. 85-89% hydrolysed). Polyvinyl alcohol is a known excipient, see USP, Ph.Eur. etc., and further explanation is considered unnecessary. The polyvinyl alcohol consists of vinyl alcohol/acetate monomers only and is not copolymerised with any other monomers. Preferably the polyvinyl alcohol is the sole polymer present in the coating. Preferably the coating further comprises titanium dioxide or talc and particularly preferably a combination of titanium dioxide and talc.

The coating preferably contains a minimum of 20 wt%, more preferably 30 wt% and most preferably 40 wt% of polyvinyl alcohol based on the total weight of the coating. The coating preferably contains a maximum of 80 wt%, more preferably 70 wt%, more preferably 60 wt% and most preferably 50 wt% of polyvinyl alcohol based on the total weight of the coating.

When present, the coating preferably contains a minimum of 10 wt%, more preferably 20 wt% and most preferably 30 wt% of titanium dioxide based on the total weight of the coating. The coating preferably contains a maximum of 60 wt%, more preferably 50 wt%, and most preferably 40 wt% of titanium dioxide based on the total weight of the coating.

When present, the coating preferably contain a minimum of 5 wt%, more preferably 10 wt% and most preferably 20 wt% of talc based on the total weight of the coating. The coating preferably contains a maximum of 50 wt%, more preferably 40 wt%, and most preferably 30 wt% of talc based on the total weight of the coating.

Where the coating contains titanium dioxide and talc the combined total is preferably a minimum of 20 wt%, more preferably 30 wt% and most preferably 40 wt% based on the total weight of the coating. The coating preferably contains a maximum of 70 wt%, more preferably 60 wt%, and most preferably 50 wt% of combined titanium dioxide and talc based on the total weight of the coating. The ratio of titanium dioxide to talc is preferably 90:10 to 10:90, more preferably 70:30 to 50:50 and most preferably about 60:40.

Olanzapine is a known compound and may be synthesised using the procedure disclosed in US 5,229,382.

The coating may be applied using known methods in the art. For example, olanzapine or a pharmaceutically acceptable salt thereof is combined with the appropriate excipients and a suspension, a dispersion or a solution of the coating composition is applied, e.g, by spraying, followed by drying the thus obtained coated composition. The pharmaceutical composition of the present invention is a coated tablet. The polyvinyl alcohol is preferably contained in the coating only.

Olanzapine formulations may be formulated in various dosage forms containing 2 to 20 mg of olanzapine and preferably 2.5, 5, 7.5, 10 or 15 mg of olanzapine.

The formulations contain pharmaceutically acceptable excipients which are well-known in the art. Suitable excipients include binders, disintegrants, fillers and lubricants.

### Examples

### Preparation Example

A coating composition was prepared containing the following components:

| Component | % | mass/g |
|---|---|---|
| Polyvinyl alcohol | 45.52 | 455.20 |
| Talc | 20.00 | 200.00 |
| Titanium dioxide | 32.00 | 320.00 |
| Xanthan gum | 0.48 | 4.80 |
| Soya lecithin | 2.00 | 20.00 |
| Total | 100.00 | 1000.00 |

### Example 1

| Ingredient | mg per tablet |
|---|---|
| Olanzapine | 10 |
| Lactose anhydrous | 233.2 |
| Microcrystalline Cellulose | 64 |
| Crospovidone | 9.6 |
| Magnesium stearate non bovine | 3.2 |

After compounding, the tablets were coated with the coating composition prepared in Preparation Example at 3.2 mg/tablet.

### Example 2

Tablets coated in accordance with Example 1 were tested for impurities at different temperatures and relative humidity.

| | % Total Impurities; 10 mg tablets stored in aluminium/aluminium blisters | |
|---|---|---|
| | Tablets from Ex. 1 | Zyprexa* |
| 0-month | LOQ** | 0.14 |
| 3-month 25°C 60% RH | LOQ | 0.28 |
| 1-month 40°C 75% RH | 0.05 | 0.21 |

| | | |
|---|---|---|
| *Commercially available tablet containing olanzapine **Below limit of quantification | | |

### Example 3

20 mg tablets were compounded with the following composition.

| Ingredient | mg per tablet |
|---|---|
| Olanzapine | 20 |
| Lactose anhydrous | 304.3 |
| Microcrystalline cellulose | 85.3 |
| Crospovidone | 12.8 |
| Magnesium stearate non bovine | 4.3 |
| | 426.7 |

The tablets were kept in an open Petri dish under visible light (VIS) according to ICH guidelines on photostability, over a period of one week. Olanzapine tablets with coating had been coated with coating formulation prepared in the Preparation Example.

| % Total impurities in 20 mg tablets | | | |
|---|---|---|---|
| | Olanzapine | | Zyprexa |
| | coated | uncoated | (coated) |
| 0-month | LOQ | LOQ | 0.07 |
| 1 week VIS | LOQ | 0.17 | 0.13 |

## Claims

1. A pharmaceutical composition which is a tablet comprising olanzapine or a pharmaceutically acceptable salt thereof, and one or more suitable pharmaceutical excipients, wherein the tablet is coated with a coating comprising polyvinyl alcohol which consists of vinyl alcohol/acetate monomers.

2. A pharmaceutical composition according to claim 1, wherein the coating further comprises titanium dioxide.

3. A pharmaceutical composition according to claim 1 or 2, wherein the coating further comprises talc.

4. A pharmaceutical composition according to any preceding claim, wherein the coating contains 20 to 80 wt% polyvinyl alcohol based on the total weight of the coating,

5. A pharmaceutical composition according to claims 2 to 4, wherein the coating contains 10 to 60 wt% titanium dioxide based on the total weight of the coating.

6. A pharmaceutical composition according to claims 3 to 5, wherein the coating contains 5 to 50 wt% talc based on the total weight of the coating.

7. A pharmaceutical composition according to any preceding claim, wherein the coating contains 20 to 80 wt% polyvinyl alcohol, 10 to 60 wt% titanium dioxide and 5 to 50 wt% talc based on the total weight of the coating.

## Patentansprüche

1. Pharmazeutische Zusammensetzung, die eine Tablette ist, die Olanzapin oder ein pharmazeutisch verträgliches Salz davon und einen oder mehrere geeignete pharmazeutische Hilfsstoffe umfasst, worin die Tablette mit einem Überzug überzogen ist, der Polyvinylalkohol umfasst, der aus Vinylalkohol/Acetat-Monomeren besteht.

2. Pharmazeutische Zusammensetzung nach Anspruch 1, worin der Überzug weiter Titandioxid umfasst.

3. Pharmazeutische Zusammensetzung nach Anspruch 1 oder 2, worin der Überzug weiter Talkum umfasst.

4. Pharmazeutische Zusammensetzung nach einem der vorstehenden Ansprüche, worin der Überzug 20 bis 80 Gew.-% Polyvinylalkohol, bezogen auf das Gesamtgewicht des Überzugs, enthält.

5. Pharmazeutische Zusammensetzung nach den Ansprüchen 2 bis 4, worin der Überzug 10 bis 60 Gew.-% Titandioxid, bezogen auf das Gesamtgewicht des Überzugs, enthält.

6. Pharmazeutische Zusammensetzung nach den Ansprüchen 3 bis 5, worin der Überzug 5 bis 50 Gew.-% Talkum, bezogen auf das Gesamtgewicht des Überzugs, enthält.

7. Pharmazeutische Zusammensetzung nach einem der vorstehenden Ansprüche, worin der Überzug 20 bis 80 Gew.-% Polyvinylalkohol, 10 bis 60 Gew.-% Titandioxid und 5 bis 50 Gew.-% Talkum, bezogen auf das Gesamtgewicht des Überzugs, enthält.

## Revendications

1. Composition pharmaceutique qui est un comprimé comprenant de l'olanzapine ou un sel pharmaceutiquement acceptable de celle-ci, et un ou plusieurs excipients pharmaceutiques appropriés, dans laquelle le comprimé est enrobé d'un enrobage comprenant de l'alcool polyvinylique qui est constitué de monomères d'alcool vinylique/acétate.

2. Composition pharmaceutique selon la revendication 1, dans laquelle l'enrobage comprend en outre du dioxyde de titane.

3. Composition pharmaceutique selon la revendication 1 ou 2, dans laquelle l'enrobage comprend en outre du talc.

4. Composition pharmaceutique selon l'une quelconque des revendications précédentes, dans laquelle l'enrobage contient 20 à 80 % en poids d'alcool polyvinylique par rapport au poids total de l'enrobage.

5. Composition pharmaceutique selon l'une quelconque des revendications 2 à 4, dans laquelle l'enrobage contient 10 à 60 % en poids de dioxyde de titane par rapport au poids total de l'enrobage.

6. Composition pharmaceutique selon l'une quelconque des revendications 3 à 5, dans laquelle l'enrobage contient 5 à 50 % en poids de talc par rapport au poids total de l'enrobage.

7. Composition pharmaceutique selon l'une quelconque des revendications précédentes, dans laquelle l'enrobage contient 20 à 80 % en poids d'alcool polyvinylique, 10 à 60 % en poids de dioxyde de titane et 5 à 50 % en poids de talc par rapport au poids total de l'enrobage.
